# EUROPÄISCHE PATENTSCHRIFT

(11) **EP 4 007 805 B1**
(45) Veröffentlichungstag und Bekanntmachung des Hinweises auf die Patenterteilung: **28.02.2024**
(21) Anmeldenummer: 20746207.8
(22) Anmeldetag: 27.07.2020
(51) Int. Cl.: C11D 17/00, E03D 9/03, A61L 2/23, A61L 9/05

(54) **REINIGUNGSMITTELFORMKÖRPER**
CLEANING PRODUCT MOLDINGS
MOULAGES DE PRODUIT DE NETTOYAGE

(30) Priorität: 01.08.2019 DE 102019211566
(43) Veröffentlichungstag der Anmeldung: 08.06.2022
(73) Patentinhaber: Henkel AG & Co. KGaA, 40589 Düsseldorf (DE)
(72) Erfinder: SCHUEMANN, Sabine, 41470 Neuss (DE); HOLDERBAUM, Thomas, 40723 Hilden (DE); MENHAM, Daniel, 40235 Düsseldorf (DE)
(86) Internationale Anmeldenummer: PCT/EP2020/071089
(87) Internationale Veröffentlichungsnummer: WO 2021/018819

(56) Entgegenhaltungen:
- EP-A1- 0 055 100
- DE-A1-102009 003 088
- DE-A1-102010 043 848
- US-A1- 2008 092 282

## Beschreibung

Die vorliegende Erfindung beschreibt einen Reinigungsmittelformkörper, dessen Verwendung sowie Verfahren unter Einsatz eines solchen Formkörpers.

WC-Reinigungsmittel stehen dem Verbraucher in einer Vielzahl unterschiedlicher Angebotsformen zur Verfügung. Neben flüssigen Reinigungsmitteln erfreuen sich dabei insbesondere die WC-Reinigungsstücke oder WC-Steine großer Beliebtheit. Diese Mittel, welche der Reinigung, Desinfektion und Beduftung von Toiletten dienen werden beispielsweise unterhalb des Beckenrands der Toilette (sogenannte Rimblocks) oder im Wasser- oder Spülkasten der Toilette (sogenannte in-tank-blocks oder cistern blocks) eingesetzt.

An diese WC-Reinigungsmittel werden seitens der Produzenten und Verbraucher eine Reihe unterschiedlicher Anforderungen gestellt, wobei seitens der Produzenten der Herstellbarkeit, Lager- und Transportfähigkeit eine besondere Bedeutung zukommt.

Ein für den Verbraucher wesentliches Produktkriterien ist neben der Produktästhetik die Produktlebensdauer, welche im Falle von WC-Reinigungsstücken durch deren Lösungs- und Abspülverhalten bestimmt wird. Um eine häufige Neudosierung des WC-Reinigungsstücks in der Toilette oder deren Spülkasten zu vermeiden, sollten diese Reinigungsstücke gleichmäßig und langanhaltend abspülen und dabei nicht durch das anströmende Wasser weggespült werden, sondern verlässlich in Toilette oder Spülkasten verbleiben.

Ästhetisch ansprechende kugelförmige WC-Reinigungsstücke mit einem guten Abspülverhalten für den Einsatz unterhalb des Beckenrands einer Toilette werden in der deutschen Patentanmeldung DE 10 2009 003 088 A1 offenbart.

Die US 2008/0092282 beschreibt einen Toilettenspender mit einer Einheitsdosis.

In der europäischen Patentanmeldung EP 055 100 A1 werden mehrphasige WC-Reinigungsblöcke unterschiedlichster geometrischer Form für den Einsatz im Spülkasten der Toilette beschrieben.

Vor diesem technischen Hintergrund bestand die Aufgabe in der Bereitstellung eines Reinigungsmittelformkörpers zur Verwendung in einem Toilettenspülkasten, welcher sich neben einer ästhetisch ansprechenden Form durch eine einfache Herstellbarkeit und gute Lager- und Transportfähigkeit auszeichnet. Darüber hinaus sollte der Formkörper herkömmlichen Mitteln hinsichtlich seines Abspülverhaltens überlegen sein und dabei auch bei unterschiedlichen Strömungsverhältnissen in verschiedenartigen Spülkästen verlässlich im Toilettenspülkasten verbleiben und die gleichmäßige Abgabe von Aktivstoffen in Wasserreservoir des Toilettenspülkasten gewährleisten.

Diese Aufgabe wurde durch einen Reinigungsmittelformkörper zur Verwendung in einem Toilettenspülkasten mit einer spezifischen Geometrie gelöst.

Ein erster Anspruchsgegenstand ist ein Reinigungsmittelformkörper zur Verwendung in einem Toilettenspülkasten, umfassend eine Auflagefläche und eine an die Auflagefläche anschließende Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt.

Die Formkörper sind vorzugsweise formstabil. "Formstabil", wie hierin verwendet, bezeichnet die Eigenschaft der Formkörper unter den für Lagerung und Transport üblichen Bedingungen ihre dreidimensionale Raumform beizubehalten, also in den für Lagerung und Transport üblichen Temperaturbereichen und unter Einwirkung der für Lagerung und Transport üblichen Kräfte weder zu desintegrieren noch irreversible Verformungen einzugehen.

Der Reinigungsmittelformkörper umfasst eine Auflage- und eine Kuppelfläche. Auflagefläche und Kuppelfläche grenzen aneinander und werden durch eine Kante voneinander getrennt. Als Auflagefläche wird eine Fläche bezeichnet, auf welcher der Formköper aufliegen kann. Hierzu umfasst die Auflagefläche mindestens drei Auflagepunkte. Der Durchmesser der Auflagefläche entspricht dem maximalen Abstand von zwei ihr zugehörigen Auflagepunkte.

Von der Auflagefläche zu unterscheiden ist die Kontaktfläche des Reinigungsmittelformkörpers. Die Kontaktfläche entspricht der Fläche mit welcher ein Reinigungsmittelformkörper, der mit seiner Auflagefläche auf einem ebenen Untergrund aufliegt, mit diesem Untergrund in Kontakt steht. Die Kontaktfläche ist im Falle einer planen Auflagefläche mit dieser identisch.

Wird der Reinigungsmittelformkörper in den Spülkasten einer Toilette eingebracht, so wird er seine räumliche Orientierung aufgrund der üblichen Strömungsverhältnisse in dem Toilettenspülkasten verändern bis er mit der Auflagefläche auf dem Boden des Spülkastens aufliegt. Aufgrund der mit dieser räumlichen Orientierung verbundenen verbesserten Haftung des Formkörpers am Spülkastenbodens wird die Bewegung des Reinigungsmittelformkörpers in dem Wasserreservoir minimiert oder ganz unterbunden. Auf diese Weise wird ein gleichmäßiges Abspülverhalten erreicht und gleichzeitig vermieden, dass der Reinigungsmittelformkörper aus dem Spülkasten ausgespült wird.

In einer bevorzugten Ausführungsform ist die Auflagefläche des Reinigungsmittelformkörpers plan. Durch die Ausgestaltung einer planen oder ebenen Auflagefläche kann das Auflage- und Haftverhalten des Formkörpers auf dem Spülkastenboden in der Regel verbessert werden. Ist die Auflagefläche plan, so ist, einen planen Spülkastenboden vorausgesetzt, bei entsprechender Orientierung des Reinigungsmittelformkörpers im Wasserreservoir die Auflagefläche identisch mit der Kontaktfläche des Reinigungsmittelformkörpers mit dem Spülkastenboden. Bevorzugte plane Auflagefläche sind kreisförmig.

Ein besonders bevorzugter Reinigungsmittelformkörper umfasst eine plane, kreisförmige Auflagefläche und eine an diese Auflagefläche angrenzende Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt.

Die zuvor beschriebenen Auflagefläche kann in unterschiedlicher Weise modifiziert werden. In einer ersten Abwandlung kann die Auflagefläche des Reinigungsmittelformkörpers Einprägungen aufweisen. Bei diesen Einprägungen kann es sich beispielsweise um geometrische Muster, bildhafte Formen, Warnhinweise, Buchstaben, Namen, Zahlen oder Markenbezeichnungen handeln. Zur besseren Kenntlichmachung können die Einprägungen und die sie umgebende Auflagefläche unterschiedliche Farben aufweisen. Beispielsweise können die Einprägungen eingefärbt sein.

Bei einer gegebenen Auflagefläche wird durch die Einprägungen die Kontaktfläche zwischen dem Reinigungsmittelformkörper und dem Spülkastenboden verringert. Mit anderen Worten ist die Auflagefläche größer als die Kontaktfläche des Reinigungsmittelformkörpers mit dem Spülkastenboden. Die Einprägungen können bis an den Rand der Auflagefläche reichen. In einer solchen Ausführungsform wird der plane Verlauf des Randes der Auflagefläche unterbrochen. Derartige Formkörper neigen bei Gebrauch zu seitlicher Unterspülung der Auflagefläche im Bereich der bis an den Rand der Auflagefläche reichenden Einprägungen und damit zu einem unregelmäßigen und wenig vorhersagbaren Abspülverhalten. In einer bevorzugten Ausführungsform sind die Einprägungen daher vollständig von Auflagefläche umgeben, reichen mit anderen Worten also nicht bis an den Rand der Auflagefläche.

Bevorzugte Reinigungsmittelformkörper, umfassend eine plane Auflagefläche und eine an die Auflagefläche anschließende Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt und wobei die Auflagefläche Einprägungen aufweist, welche nicht bis an den Rand der Auflagefläche reichen.

In einer alternativen Ausführungsform weist die Auflagefläche plane und konkave Abschnitte auf. In dieser Ausführungsform ist nicht die gesamte Auflagefläche konkav gekrümmt. Vielmehr sind Teilbereiche oder Abschnitte der Auflagefläche konkav gekrümmt während andere Abschnitte oder Teilbereiche plan sind. Ein Beispiel für eine derartige Oberflächenstruktur sind zwei, drei, vier oder mehr zufällig oder regelmäßig angeordnete konkave Versenkungen im Bereich der Auflagefläche.

Reichen die konkaven Abschnitte bis an den Rand der Auflagefläche so wird der plane Verlauf des Randes der Auflagefläche unterbrochen. Die Kante zwischen Auflagefläche und Kuppelfläche verläuft bei derartigen Formkörpern wellenförmig. Reinigungsmittelformkörper, bei denen die Kante zwischen Auflagefläche und Kuppelfläche wellenförmig verläuft, sind aus ästhetischen Gesichtspunkten bevorzugt.

Wie bereits im Falle der Reinigungsmittelformkörper mit Einprägungen beschrieben neigen jedoch auch diese Formkörper bei Gebrauch zu seitlicher Unterspülung der Auflagefläche im Bereich der bis an den Rand der Auflagefläche reichenden konkaven Versenkungen und damit zu einem unregelmäßigen und wenig vorhersagbaren Abspülverhalten. In einer bevorzugten Ausführungsform sind die konkaven Abschnitte daher vollständig von Auflagefläche umgeben, reichen mit anderen Worten also nicht bis an den Rand der Auflagefläche.

In Bezug auf die Haftung des Formkörpers am Spülkastenboden, das Abspülverhalten und den Verbleib des Reinigungsmittelformkörpers in dem Spülkasten haben sich solche Reinigungsmittelformkörper als vorteilhaft erwiesen, bei denen die Kante zwischen der Auflagefläche und der Kuppelfläche plan ist.

In einer Fortführung der zuvor beschriebenen Ausführungsform ist die Auflagefläche vollständig konkav ausgestaltet. Die Kontaktfläche zwischen dem Reinigungsmittelformkörper und dem Boden wird mit anderen Worten allein durch den Randbereich der Auflagefläche gebildet, welche die konkave Einwölbung der Auflagefläche umgibt.

Bei einer räumlich nach unten orientierten Auflagefläche schließt sich oberhalb an die Auflagefläche die Kuppelfläche an. Die Kuppelfläche kann in unterschiedlicher Weise ausgestaltet werden, sofern das für den Reinigungsmittelformkörper kennzeichnende Verhältnis des maximalen Durchmessers der Auflagefläche zu dem maximalen Durchmesser einer zu der Auflagefläche parallelen Querschnittsfläche der Kuppelfläche 1:20 bis 4:5 eingehalten wird.

Hinsichtlich der Haftung des Formkörpers am Spülkastenboden, das Abspülverhalten und den Verbleib des Reinigungsmittelformkörpers in dem Spülkasten haben sich solche Reinigungsmittelformkörper als besonders vorteilhaft erwiesen, bei denen Reinigungsmittelformkörper ein Verhältnis des maximalen Durchmessers der Auflagefläche zu dem maximalen Durchmesser einer zu der Auflagefläche parallelen Querschnittsfläche der Kuppelfläche 1:15 bis 2:3, vorzugsweise 1:10 bis 1:2 aufweist.

Einer ersten Ausführungsform ist die Kuppelfläche konvex polyedrisch ausgestaltet. Ein solcher Reinigungsmittelformkörper weist also neben der Auflagefläche eine Vielzahl weiterer Grenzflächen auf. Im Falle einer konvex polyedrischen Kuppelfläche ist die Auflagefläche größer als jede der weiteren Grenzflächen im Bereich der Kuppelfläche. Vorzugsweise beträgt das Verhältnis der Fläche der Auflagefläche zur Fläche der größten weiteren Grenzfläche der konvex polyedrischen Kuppel mindestens 2:1, vorzugsweise 2:1 bis 80:1 und insbesondere 4:1 bis 60:1.

Ein weiterer Anmeldungsgegenstand ist ein Reinigungsmittelformkörper, umfassend eine Auflagefläche und eine an die Auflagefläche anschließende konvex polyedrische Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt und Verhältnis der Fläche der Auflagefläche zur Fläche der größten weiteren Grenzfläche der konvex polyedrischen Kuppel mindestens 2:1, vorzugsweise 2:1 bis 80:1 und insbesondere 4:1 bis 60:1 beträgt.

Für die räumliche Orientierung des Formkörpers in dem Spülkasten und das Abspülverhalten des Formkörpers hat es sich als vorteilhaft erwiesen, die Kuppelfläche wenigstens anteilsweise abzurunden.

Für die räumliche Orientierung des Formkörpers in dem Spülkasten und die Vorhersagbarkeit des Abspülverhaltens des Formkörpers hat es sich weiterhin als vorteilhaft erwiesen, wenn der Formkörper orthogonal zur Auflagefläche eine Symmetrieachse aufweist.

Besonders bevorzugt sind Reinigungsmittelformkörper, welche parallel zur Auflagefläche eine kreisförmige oder ellipsoide Querschnittsfläche, vorzugsweise eine kreisförmige Querschnittsfläche aufweist. In gleicher Weise bevorzugt sind Reinigungsmittelformkörper, welche orthogonal zur Auflagefläche einen Querschnitt in Form eines Kreisbogens oder eines Ellipsenbogens, vorzugsweise in Form eines Kreisbogens aufweisen.

Abgerundete, beispielsweise sphärisch oder ellipsoid ausgestaltete Kuppelflächen haben gegenüber den weiter oben beschriebenen konvex polyedrischen Kuppelflächen Vorteile in Bezug auf ihr Abspülverhalten, insbesondere in Bezug auf das gleichmäßige und langanhaltende Abspülverhalten und dessen Vorhersagbarkeit in Toilettenspülkästen unterschiedlicher Bauart und mit unterschiedlichen Strömungsverhältnissen.

Die Reinigungsmittelformkörper mit konvex polyedrischen Kuppelfläche wiederum sind in Bezug auf ihr Abspülverhalten und dessen Vorhersagbarkeit den hexaedrischen Reinigungsmittelformkörpern überlegen.

Zusammenfassend sind solche Reinigungsmittelformkörper besonders bevorzugt, welche eine plane, kreisförmige Auflagefläche und eine an die Auflagefläche anschließende Kuppelfläche umfassen, wobei
- das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt;
- der Formkörper parallel zur Auflagefläche eine kreisförmige Querschnittsfläche aufweist;
- der Formkörper orthogonal zur Auflagefläche einen Querschnitt in Form eines Kreisbogens aufweist.

Mit anderen Worten sind Reinigungsmittelformkörper besonders bevorzugt, welche in Form eines Kugelsegments vorliegen, wobei das Verhältnis des maximalen Durchmessers der Grundfläche zu dem maximalen Durchmesser einer zu der Grundfläche parallelen Querschnittsfläche der Kuppel 1:20 bis 4:5 beträgt.

Wie bereits im Zusammenhang mit der Auflagefläche beschrieben kann der Reinigungsmittelformkörper auch im Bereich der Kuppelfläche Einprägungen aufweisen. Bei diesen Einprägungen kann es sich ebenfalls um geometrische Muster, bildhafte Formen, Warnhinweise, Buchstaben, Namen, Zahlen oder Markenbezeichnungen handeln. Zur besseren Kenntlichmachung können die Einprägungen und die sie umgebende Auflagefläche unterschiedliche Farben aufweisen. Beispielsweise können die Einprägungen eingefärbt sein.

Die zuvor beschriebenen Reinigungsmittelformkörper entfalten ihre vorteilhaften technischen Eigenschaften insbesondere beim Eintrag in den Spülkasten einer Toilette. Die Verwendung der in dieser Beschreibung offenbarten Reinigungsmittelformkörper zur Reinigung oder Beduftung des Wassers in einem Toilettenspülkasten ist daher ein weiterer Gegenstand dieser Anmeldung.

Bevorzugt werden insbesondere solche Verwendungen, bei denen der Formkörper für mindestens 40, vorzugsweise mindestens 80 und insbesondere mindestens 200 Spülgängen in dem Toilettenspülkasten verbleibt.

Die im Zusammenhang mit dem Reinigungsmittelformkörper weiter oben und nachfolgend offenbarten spezifischen technischen Merkmale gelten mutatis mutandis auch für deren Verwendung. Zur Vermeidung von Wiederholungen wird an dieser Stelle auf die dortigen Ausführungen verwiesen.

Wie zuvor ausgeführt sind die Formkörper vorzugsweise dazu geeignet, für mindestens 40, vorzugsweise mindestens 80 und insbesondere mindestens 200 Spülgänge in dem Toilettenspülkasten zu verbleiben.

Vor dem Hintergrund der für die Reinigungsmittelformkörper angestrebten Lebensdauer weisen diese vorzugsweise ein Gewicht von 10 bis 160 g, vorzugsweise von 15 bis 120 g und insbesondere von 20 bis 90 g auf.

Die Dichte der Reinigungsmittelformkörper beträgt vorzugsweise 1,2 bis 2,0 g/cm³, besonders bevorzugt 1,4 bis 1,9 g/cm³ und insbesondere 1,5 bis 1,8 g/cm³. Diese Dichtebereiche habe sich in Kombination mit der spezifischen Raumform der Formkörper im Hinblick auf die Orientierung und den Verbleib im Spülkasten als besonders vorteilhaft erwiesen. Aus den gleichen Gründen weist die Auflagefläche der Reinigungsmittelformkörper vorzugsweise einen maximalen Durchmesser von 3 bis 20 mm, vorzugsweise von 4 bis 18 mm und insbesondere von 5 bis 15 mm auf.

Das Volumen der Reinigungsmittelformkörper liegt bevorzugt im Bereich von 8 bis 100 ml, vorzugsweise von 12 bis 80 ml und insbesondere von 15 bis 60 ml.

Bevorzugte Reinigungsmittelformkörper weisen eine Auflagefläche und eine an die Auflagefläche anschließende Kuppelfläche auf, wobei
- das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt;
- der Reinigungsmittelformkörper ein Gewicht von 20 bis 90 g aufweist und
- die Dichte des Reinigungsmittelformkörpers 1,5 bis 1,8 g/cm³ beträgt.

Die Reinigungsmittelformkörper können ein- oder mehrphasig sein. Aufgrund ihrer einfacheren Herstellbarkeit sind einphasige Formkörper bevorzugt.

Als wesentliche Bestandteile enthalten die Reinigungsmittelformkörper vorzugsweise neben einem wasserdispergierbaren Trägermaterial einen oder mehrere Aktivstoffe. Bevorzugt ist es insbesondere, wenn der Reinigungsmittelformkörper
a) 20 bis 90 Gew.-% wasserdispergierbares Trägermaterial
b) 10 bis 80 Gew.-% Aktivstoffe
umfasst. Als Aktivstoffe werden dabei vorzugsweise Tenside, Duftstoffe und Farbstoffe eingesetzt. Ganz besonders bevorzugt ist eine Wirkstoffkombination, umfassend Tensid, Duftstoff und Farbstoff.

Die Merkmale einiger bevorzugter Reinigungsmittelformkörper kann den folgenden Tabellen entnommen werden (Angaben für das Trägermaterial und die Aktivstoffe in Gew.-% bezogen auf das Gesamtgewicht des Mittels sofern nicht anders angegeben).

| | Körper 1 | Körper 2 | Körper 3 | Körper 4 | Körper 5 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |

| | Körper 6 | Körper 7 | Körper 8 | Körper 9 | Körper 10 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |

| | Körper 11 | Körper 12 | Körper 13 | Körper 14 | Körper 15 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Auflagefläche | kreisförmig, plan | | | | |

| | Körper 16 | Körper 17 | Körper 18 | Körper 19 | Körper 20 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Auflagefläche | kreisförmig, plan | | | | |

| | Körper 21 | Körper 22 | Körper 23 | Körper 24 | Körper 25 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Auflagefläche | kreisförmig, plan | | | | |
| Querschnittsfläche *** | kreisförmig | | | | |

| | Körper 26 | Körper 27 | Körper 28 | Körper 29 | Körper 30 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Auflagefläche | kreisförmig, plan | | | | |
| Querschnittsfläche *** | kreisförmig | | | | |

| | Körper 31 | Körper 32 | Körper 33 | Körper 34 | Körper 35 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Auflagefläche | kreisförmig, plan | | | | |
| Querschnittsfläche *** | kreisförmig | | | | |
| Querschnittsfläche **** | Kreisbogen | | | | |

| | Körper 36 | Körper 37 | Körper 38 | Körper 39 | Körper 40 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Auflagefläche | kreisförmig, plan | | | | |
| Querschnittsfläche *** | kreisförmig | | | | |
| Querschnittsfläche **** | Kreisbogen | | | | |

| | Körper 41 | Körper 42 | Körper 43 | Körper 44 | Körper 45 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Wasserdispergierbares Trägermaterial | 20 bis 90 | | | | |
| Aktivstoffe | 10 bis 80 | | | | |

| | Körper 46 | Körper 47 | Körper 48 | Körper 49 | Körper 50 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Wasserdispergierbares Trägermaterial | 20 bis 90 | | | | |
| Aktivstoffe | 10 bis 80 | | | | |

| | Körper 51 | Körper 52 | Körper 53 | Körper 54 | Körper 55 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Wasserdispergierbares Trägermaterial | 20 bis 90 | | | | |
| Aktivstoffe | 10 bis 80 | | | | |
| Auflagefläche | kreisförmig, plan | | | | |

| | Körper 56 | Körper 57 | Körper 58 | Körper 59 | Körper 60 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Wasserdispergierbares Trägermaterial | 20 bis 90 | | | | |
| Aktivstoffe | 10 bis 80 | | | | |
| Auflagefläche | kreisförmig, plan | | | | |

| | Körper 61 | Körper 62 | Körper 63 | Körper 64 | Körper 65 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Wasserdispergierbares Trägermaterial | 20 bis 90 | | | | |
| Aktivstoffe | 10 bis 80 | | | | |
| Auflagefläche | kreisförmig, plan | | | | |
| Querschnittsfläche *** | kreisförmig | | | | |

| | | | | | |
|---|---|---|---|---|---|
| | Körper 66 | Körper 67 | Körper 68 | Körper 69 | Körper 70 |
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Wasserdispergierbares Trägermaterial | 20 bis 90 | | | | |
| Aktivstoffe | 10 bis 80 | | | | |
| Auflagefläche | kreisförmig, plan | | | | |
| Querschnittsfläche *** | kreisförmig | | | | |

| | Körper 71 | Körper 72 | Körper 73 | Körper 74 | Körper 75 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Dichte (g/cm³) | 1,2 bis 2,0 | 1,4 bis 1,9 | 1,4, bis 1,9 | 1,5 bis 1,8 | 1,5 bis 1,8 |
| Wasserdispergierbares Trägermaterial | 20 bis 90 | | | | |
| Aktivstoffe | 10 bis 80 | | | | |
| Auflagefläche | kreisförmig, plan | | | | |
| Querschnittsfläche *** | kreisförmig | | | | |
| Querschnittsfläche **** | Kreisbogen | | | | |

| | Körper 76 | Körper 77 | Körper 78 | Körper 79 | Körper 80 |
|---|---|---|---|---|---|
| Verhältnis * | 1:20 bis 4:5 | 1:20 bis 4:5 | 1:15 bis 2:3 | 1:15 bis 2:3 | 1:10 bis 1:2 |
| Gewicht (g) | 10 bis 160 | 15 bis 120 | 15 bis 120 | 15 bis 120 | 20 bis 90 |
| Durchmesser (mm) ** | 3 bis 20 | 4 bis 18 | 4 bis 18 | 5 bis 15 | 5 bis 15 |
| Wasserdispergierbares Trägermaterial | 20 bis 90 | | | | |
| Aktivstoffe | 10 bis 80 | | | | |
| Auflagefläche | kreisförmig, plan | | | | |
| Querschnittsfläche *** | kreisförmig | | | | |
| Querschnittsfläche **** | Kreisbogen | | | | |

| | | | | | |
|---|---|---|---|---|---|
| * Verhältnis des maximalen Durchmessers der Auflagefläche zu dem maximalen Durchmesser einer zu der Auflagefläche parallelen Querschnittsfläche der Kuppelfläche ** maximaler Durchmesser der Auflagefläche *** Querschnittsfläche parallel zur Auflagefläche **** Querschnittsfläche orthogonal zur Auflagefläche | | | | | |

Zur Nutzung der zuvor beschriebenen Reinigungsmittelformkörper werden diese in den Spülkasten einer Toilette eingebracht und es wird nachfolgend die Spülung der Toilette betätigt.

Ein Verfahren zur Reinigung oder Beduftung des Wassers in einem Toilettenspülkasten, umfassend die Schritte
a) Einbringen eines Reinigungsmittelformkörpers nach einem der Punkte 1 bis 19 in das Wasserreservoir eines Toilettenspülkastens;
b) Betätigen der Spülung unter wenigstens anteilsweisem Austausch des Wasserreservoris in dem Toilettenspülkasten
ist ein weiterer Gegenstand dieser Anmeldung. Der in diesem Verfahren eingesetzte Toilettenspülkasten weist vorzugsweise einen ebenen Boden auf. Im Rahmen dieses Verfahrens kann ein einzelner Reinigungsmittelformkörper oder eine Mehrzahl von Reinigungsmittelformkörpern gleichzeitig in das Wasserreservoir des Toilettenspülkastens eingebracht werden. Bevorzugt ist es jedoch, einen einzelnen Reinigungsmittelformkörper in das Wasserreservoir einzubringen, welcher dort für mindestens 40, vorzugsweise mindestens 80 und insbesondere mindestens 200 Spülgänge verbleibt.

Wie bereits zuvor im Zusammenhang mit der erfindungsgemäßen Verwendung ausgeführt, gelten die im Zusammenhang mit dem Reinigungsmittelformkörper weiter oben offenbarten spezifischen technischen Merkmale mutatis mutandis auch für das beschriebene Verfahren. Zur Vermeidung von Wiederholungen wird auch an dieser Stelle auf die dortigen Ausführungen verwiesen.

Die folgenden Abbildungen verdeutlichen Ausführungsformen der vorliegenden Erfindung:
- Fig 1a: Reinigungsmittelformkörper, dessen orthogonal zur Auflagefläche verlaufende Querschnitt die Form eines Kreisbogens aufweist.
- Fig 1b: Reinigungsmittelformkörper, dessen orthogonal zur Auflagefläche verlaufende Querschnitt die Form eines Ellipsenbogens aufweist.
- Fig 2: Verwendung eines Reinigungsmittelformkörper gemäß dem Stand der Technik
- Fig 3: Verwendung eines Reinigungsmittelformkörper gemäß der Erfindung

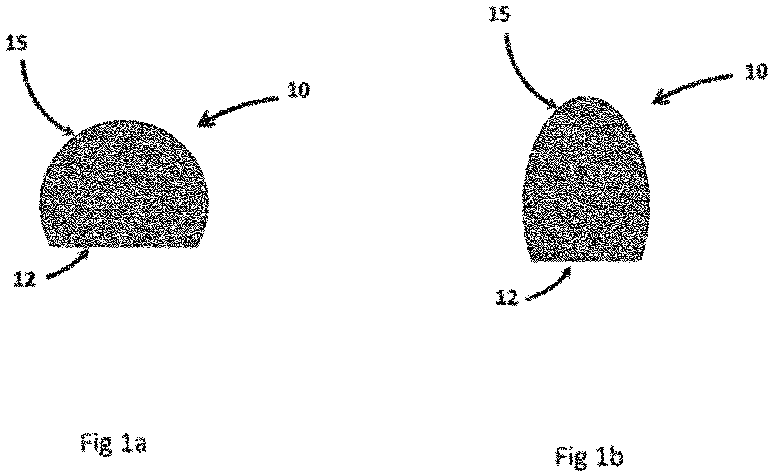

Fig 1a zeigt einen Reinigungsmittelformkörper 10 gemäß der Erfindung. Der Reinigungsmittelformkörper 10 weist eine Auflagefläche 12 und eine Kuppelfläche 15 auf. Die Kuppelfläche ist kugelförmig ausgebildet.

Fig 1b zeigt einen Reinigungsmittelformkörper 10 gemäß der Erfindung. Der Reinigungsmittelformkörper 10 weist eine Auflagefläche 12 und eine Kuppelfläche 15 auf. Die Kuppelfläche ist ellipsoid ausgebildet.

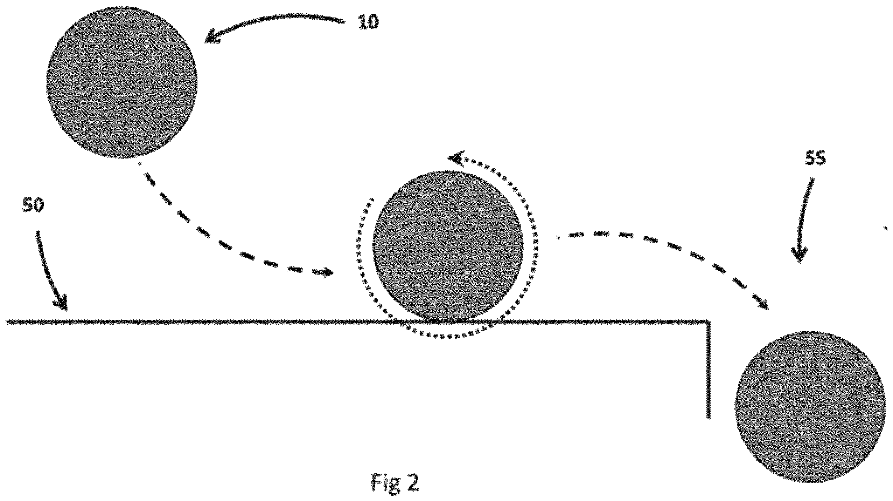

Fig 2 zeigt die Verwendung eines Reinigungsmittelformkörper 10 gemäß dem Stand der Technik.

Der Reinigungsmittelformkörper 10 ist als Kugel ausgebildet. Der Reinigungsmittelformkörper 10 weist keine Auflagefläche 12 auf.

Wird der Reinigungsmittelformkörper 10 in einen Toilettenspülkasten eingebracht kann sich der Reinigungsmittelformkörper 10 im Spülkasten im Toilettenspülkasten in dem Wasserreservoir insbesondere aber auf dem Boden 50 des Toilettenspülkastens bewegen. Der Reinigungsmittelformkörper 10 kann durch diese Bewegung, beispielsweise eine Rollbewegung, durch den Abfluss 55 des Toilettenspülkastens ausgespült werden.

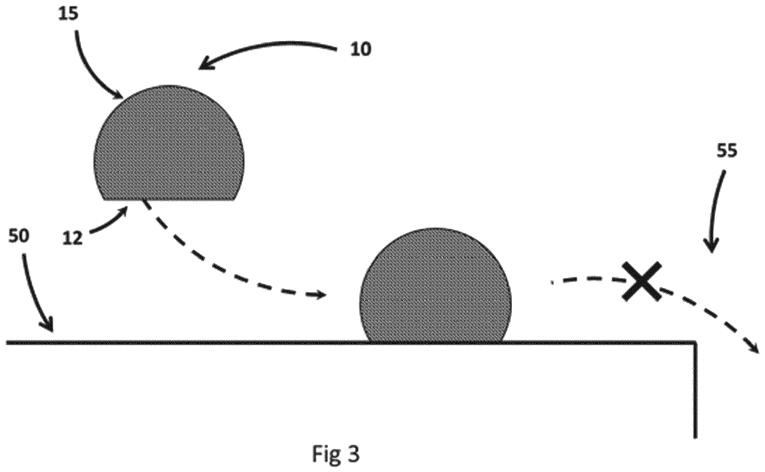

Fig 3 zeigt die Verwendung eines Reinigungsmittelformkörper 10 gemäß der Erfindung. Der Reinigungsmittelformkörper 10 weist insbesondere eine Auflagefläche 12 auf.

Dadurch, dass der erfindungsgemäße Reinigungsmittelformkörper 10 neben einer Kuppelfläche 15 eine Auflagefläche 12 aufweist, kann der Reinigungsmittelformkörper 10 im Spülkasten mit der Auflagefläche 12 am Boden 50 des Toilettenspülkastens aufliegen, ohne sich im Toilettenspülkasten zu bewegen. Ein ungleichmäßiges Abspülverhalten wird vermieden. Ferner wird vermieden, dass der Reinigungsmittelformkörper 10 durch eine Rollbewegung aus dem Spülkasten durch den Ausfluss 55 des Toilettenspülkastens ausgespült wird. Vorteilhaft wurde ferner beobachtet, dass der Reinigungsmittelformkörper 10, sofern dieser nicht schon nach Einbringen in das Wasserreservoir und Absinken auf den Boden 50 des Toilettenspülkastens mit der Auflagefläche 12 auf den Boden 50 trifft, sich bei den üblichen Strömungsverhältnissen im Toilettenspülkasten durch Rollbewegung in eine Position bewegt, bei der die Auflagefläche 12 auf dem Boden aufliegt. Diese Rollbewegung wird insbesondere durch eine Ausgestaltung des Formkörpers begünstigt, bei welcher dieser orthogonal zur Auflagefläche einen Querschnitt in Form eines Kreisbogens oder eines Ellipsenbogens, vorzugsweise in Form eines Kreisbogens aufweist.

Sobald der Reinigungsmittelformkörper 10 mit der Auflagefläche 12 auf dem Boden 50 aufliegt, kann der Reinigungsmittelformkörper 10 nicht mehr durch eine Rollbewegung aus dem Spülkasten durch den Ausfluss 55 herausgespült werden.

### Bezugszeichenliste:

10 Reinigungsmittelformkörper
12 Auflagefläche
15 Kuppelfläche
50 Boden Toilettenspülkasten
55 Abfluss Toilettenspülkasten

## Patentansprüche

1. Reinigungsmittelformkörper, zur Verwendung in einem Toilettenspülkasten umfassend eine Auflagefläche und eine an die Auflagefläche anschließende Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt.

2. Reinigungsmittelformkörper nach Anspruch 1, umfassend eine plane, kreisförmige Auflagefläche und eine an diese Auflagefläche angrenzende kugelförmige Kuppelfläche, wobei das Verhältnis des maximalen Durchmessers der Auflagefläche zu dem maximalen Durchmesser einer zu der Auflagefläche parallelen Querschnittsfläche der Kuppelfläche 1:20 bis 4:5 beträgt.

3. Reinigungsmittelformkörper nach einem der vorherigen Ansprüche, wobei das Verhältnis des maximalen Durchmessers der Auflagefläche zu dem maximalen Durchmesser einer zu der Auflagefläche parallelen Querschnittsfläche der Kuppelfläche 1:15 bis 2:3, vorzugsweise 1:10 bis 1:2 beträgt.

4. Reinigungsmittelformkörper nach einem der vorherigen Ansprüche, wobei der Formkörper geeignet ist, für mindestens 40, vorzugsweise mindestens 80 und insbesondere mindestens 200 Spülgängen in dem Toilettenspülkasten zu verbleiben.

5. Reinigungsmittelformkörper nach einem der vorherigen Ansprüche, wobei die Auflagefläche einen maximalen Durchmesser von 3 bis 20 mm, vorzugsweise von 4 bis 18 mm und insbesondere von 5 bis 15 mm aufweist.

6. Reinigungsmittelformkörper nach einem der vorherigen Ansprüche, wobei der Formkörper ein Gewicht von 10 bis 160 g, vorzugsweise von 15 bis 120 g und insbesondere von 20 bis 90 g aufweist.

7. Reinigungsmittelformkörper nach einem der vorherigen Ansprüche, wobei der Formkörper eine Dichte von 1,2 bis 2,0 g/cm³, besonders bevorzugt von 1,4 bis 1,9 g/cm³ und insbesondere von 1,5 bis 1,8 g/cm³ aufweist.

8. Reinigungsmittelformkörper nach einem der vorherigen Ansprüche, umfassend
a) 20 bis 90 Gew.-% wasserdispergierbares Trägermaterial;
b) 10 bis 80 Gew.-% Aktivstoffe.

9. Verwendung eines Reinigungsmittelformkörpers nach einem der vorherigen Ansprüche zur Reinigung oder Beduftung des Wassers in einem Toilettenspülkasten.

## Claims

1. Cleaning agent molded body for use in a toilet cistern comprising a support surface and a dome surface adjoining the support surface, the ratio of the maximum diameter of the support surface to the maximum diameter of a cross-sectional surface of the dome surface parallel to the support surface being 1:20 to 4:5.

2. Cleaning agent molded body according to claim 1, comprising a flat, circular bearing surface and a spherical dome surface adjacent to this bearing surface, the ratio of the maximum diameter of the bearing surface to the maximum diameter of a cross-sectional surface of the dome surface parallel to the bearing surface being 1:20 to 4:5.

3. Cleaning agent molded body according to one of the preceding claims, wherein the ratio of the maximum diameter of the bearing surface to the maximum diameter of a cross-sectional surface of the dome surface parallel to the bearing surface is 1:15 to 2:3, preferably 1:10 to 1:2.

4. Cleaning agent molded body according to one of the preceding claims, wherein the molded body is suitable to remain in the toilet cistern for at least 40, preferably at least 80 and in particular at least 200 flushing cycles.

5. Cleaning agent molded body according to one of the preceding claims, wherein the contact surface has a maximum diameter of 3 to 20 mm, preferably of 4 to 18 mm and in particular of 5 to 15 mm.

6. Cleaning agent molded article according to one of the preceding claims, wherein the molded article has a weight of 10 to 160 g, preferably of 15 to 120 g and in particular of 20 to 90 g.

7. Cleaning agent molded article according to one of the preceding claims, wherein the molded article has a density of from 1.2 to 2.0 g/cm³, particularly preferably from 1.4 to 1.9 g/cm³ and in particular of 1.5 to 1.8 g/cm³.

8. cleaning agent molded article according to any one of the preceding claims, comprising
a) 20 to 90 % by weight of water-dispersible carrier material;
b) 10 to 80 % by weight of active substances.

9. use of a cleaning agent shaped body according to one of the preceding claims for cleaning or scenting the water in a toilet cistern.

## Revendications

1. Corps moulé de détergent destiné à être utilisé dans un réservoir de chasse d'eau comprenant une surface de support et une surface de dôme contiguë à la surface de support, dans lequel le rapport du diamètre maximal de la surface de support au diamètre maximal d'une surface de section transversale de la surface de dôme parallèle à la surface de support est de 1:20 à 4:5.

2. Corps moulé de détergent selon la revendication 1, comprenant une surface d'appui circulaire plane et une surface d'accouplement sphérique adjacente à cette surface d'appui, le rapport entre le diamètre maximal de la surface d'appui et le diamètre maximal d'une surface de section transversale de la surface d'accouplement parallèle à la surface d'appui étant de 1:20 à 4:5.

3. Corps moulé de détergent selon l'une des revendications précédentes, dans lequel le rapport entre le diamètre maximal de la surface d'appui et le diamètre maximal d'une surface de section transversale de la surface d'accouplement parallèle à la surface d'appui est de 1:15 à 2:3, de préférence de 1:10 à 1:2.

4. Corps moulé détergent selon l'une quelconque des revendications précédentes, ledit corps moulé étant apte à rester dans le réservoir de chasse d'eau pendant au moins 40, de préférence au moins 80 et en particulier au moins 200 cycles de chasse d'eau.

5. Corps moulé de détergent selon l'une des revendications précédentes, dans lequel la surface d'appui présente un diamètre maximal de 3 à 20 mm, de préférence de 4 à 18 mm et en particulier de 5 à 15 mm.

6. Corps moulé de détergent selon l'une des revendications précédentes, dans lequel le corps moulé présente un poids de 10 à 160 g, de préférence de 15 à 120 g et en particulier de 20 à 90 g.

7. Corps moulé de détergent selon l'une des revendications précédentes, dans lequel le corps moulé présente une densité de 1,2 à 2,0 g/cm³, de préférence de 1,4 à 1,9 g/cm³ et en particulier de 1,5 à 1,8 g/cm³.

8. Corps moulé de détergent selon l'une quelconque des revendications précédentes, comprenant
a) 20 à 90 % en poids de matériau support dispersible dans l'eau;
b) 10 à 80 % en poids de substances actives.

9. Utilisation d'un corps moulé détergent selon l'une des revendications précédentes pour nettoyer ou parfumer l'eau d'un réservoir de chasse d'eau.
